# EUROPEAN PATENT APPLICATION

(11) **EP 2 682 094 A1**
(43) Date of publication of application: **08.01.2014**
(21) Application number: 12752276.1
(22) Date of filing: 22.02.2012
(51) Int. Cl.: A61K 8/06, A61K 8/34, A61K 8/39, A61K 8/86, A61K 8/92, A61K 9/107, A61K 47/10, A61K 47/34, A61Q 19/00

(54) **MANUFACTURING METHOD FOR O/W EMULSION COMPOSITION**

(30) Priority: 01.03.2011 JP 2011043593
(71) Applicant: Shiseido Company, Ltd., Tokyo 104-0061 (JP)
(72) Inventor: UYAMA, Makoto, Yokohama-shi Kanagawa 224-8558 (JP); MIYAHARA, Reiji, Yokohama-shi Kanagawa 224-8558 (JP); ARAKI, Hidefumi, Yokohama-shi Kanagawa 224-8558 (JP)
(74) Representative: Lippert, Stachow & Partner
(86) International application number: PCT/JP2012/054239
(87) International publication number: WO 2012/117909

(57) **Abstract**

The present invention provides a production method that can easily produce an O/W emulsion composition without using a special cooling apparatus and be economical. The production method of an O/W emulsion composition comprising the preparation of an emulsified part that is an O/W emulsion by emulsifying, at a temperature of 70 °C or higher, an oil phase comprising (A) a mono-branched fatty acid POE (0-60) glycerin ester, (B) a linear higher alcohol having 16 or more carbon atoms capable to form an α-gel in water with said (A), and (C) an oil component, and a part of an aqueous phase (a first aqueous phase) comprising (D) water; and the cooling of this emulsified part by mixing with stirring the remaining main aqueous phase (a second aqueous phase) at 10 to 35 °C, wherein an aqueous solvent in the emulsified part is 15 mass % or less.

## Description

### RELATED APPLICATIONS

This application claims the priority of Japanese Patent Application No. 2011-43593 filed on March 1, 2011, which is incorporated herein by reference.

### FIELD OF THE INVENTION

The present invention relates to a production method of an O/W emulsion composition, and in particular, relates to a production method that is excellent in simplicity and economy.

### BACKGROUND OF THE INVENTION

In the past, in order to maintain the emulsion stability of external skin preparations such as cosmetics, quasi-drugs, and pharmaceuticals, an O/W emulsion composition with the use of an α-gel, which is formed from a higher aliphatic alcohol and a polyoxyethylene-type nonionic surfactant, has been used and it is especially known for use in cosmetics. As the preparation method of such an O/W emulsion composition, the following method has been used: a moisturizer and a hydrophilic polyoxyethylene-type nonionic surfactant are dissolved in water and heated to about 70 °C to prepare a aqueous phase; an oil component and a higher alcohol, as essential components, were homogeneously mixed at about 70 °C to prepare an oil phase; the oil phase was added and emulsified into the aqueous phase while being stirred with a homogenizer; and the obtained emulsion is rapidly cooled with a cooling machine such as an Onlator to about 35 °C (for example, refer to Non-patent Literature 1).

However, the conventional method, wherein the cooling is carried out after emulsification at about 70 °C, is wasteful because of the energy necessary for heating and for the use of a heat exchanger. In addition, the environmental burden is high because a large amount of water is used for washing a cooling machine such as an Onlator after use.

Accordingly, the development of a method by which an O/W emulsion composition having a comparable feeling in use to that of the conventional one can be economically and easily produced without the use of a conventional cooling device such as an Onlator has been awaited.

In patent literature 1, a production method of a fine O/W emulsion external preparation having the emulsion particle size of 50 to 500 nm, wherein the method comprises a W/O emulsion preparation process in which the W/O emulsion is prepared by mixing with stirring, at 70 to 80 °C, (A) a hydrophilic nonionic surfactant, (B) a linear higher alcohol having 16 or more carbon atoms, (C) an oil component, (D) a water-soluble aqueous solvent wherein the critical micelle concentration (cmc) of the above-described hydrophilic nonionic surfactant in the aqueous solvent is higher than that in water, and (E) water in the amount of 5 to 25 weight % of the total amount of (A) to (E); and a fine O/W emulsion preparation process in which the W/O emulsion is inverted to a fine O/W emulsion by the addition, while mixing with stirring, of (F) water or an aqueous formulation at 10 to 35 °C into the W/O emulsion is described.

According to the method of patent literature 1, a fine O/W emulsion, which was difficult to prepare by the conventional method in which a cooler such as an Onlator is used, can be easily produced with low energy without the use of an Onlator. In addition, the fine O/W emulsion obtained by the method of patent literature 1 has more refreshing and richer usability than the O/W emulsion of the identical formulation and produced by the conventional method with the use of a cooler such as an Onlator.

However, the method of patent literature 1 is a method in which a W/O emulsion is once prepared with a specific composition and a fine O/W emulsion is obtained by phase inversion; therefore, the composition was sometimes limited. In patent literature 1, no O/W emulsion production method, without passing through the phase inversion from the W/O emulsion, is described.

Patent Literature 1: International unexamined patent publication No. 2010/082602

Non-patent Literature 1: "Chemistry and Application of Surface Activity" (in Japanese) written by Manabu Senoo, Dainippon Tosho Co., Ltd., 1995; p 159-160.

### SUMMARY OF THE INVENTION

### PROBLEM TO BE SOLVED BY THE INVENTION

The present invention was made in view of the above-described situation of the background art, and the problem to be solved is to provide a simple and economical production method of an O/W emulsion composition.

### MEANS TO SOLVE THE PROBLEM

In view of the above-described problems, the present inventors have diligently studied; as a result, the present inventors have found that if a specific nonionic surfactant and a linear higher alcohol having 16 or more carbon atoms and capable to form an α-gel with the nonionic surfactant in water is used, and the amount of an aqueous solvent is controlled, an O/W emulsion composition having a comparable feeling in use to that by the conventional method, in which an Onlator etc. is used, can be produced by using a part of the aqueous phase for cooling, thus leading to the completion of the present invention.

That is, the production method of an O/W emulsion composition of the present invention comprises the preparation of an emulsified part that is an O/W emulsion by emulsifying, at a temperature of 70 °C or higher, an oil phase comprising
(A) a mono-branched fatty acid POE (0-60) glycerin ester,
(B) a linear higher alcohol having 16 or more carbon atoms capable to form an α-gel in water with said (A), and
(C) an oil component,
and a part of an aqueous phase (a first aqueous phase) comprising (D) water;
and the cooling of this emulsified part by mixing with stirring the remaining main aqueous phase (a second aqueous phase) at 10 to 35 °C, wherein an aqueous solvent in the emulsified part is 15 mass % or less.

It is preferable in the production method that the aqueous solvent is a dihydric to trihydric polyol.

It is preferable in the production method that the main aqueous phase is 50 to 75 mass % of the total O/W emulsion composition.

It is preferable in the production method according to any of the above-described methods that the sum of (A) a mono-branched fatty acid POE (0-60) glycerin ester and (B) a linear higher alcohol having 16 or more carbon atoms is 0.5 to 10 parts by mass with respect to 10 parts by mass of (C) an oil component.

### EFFECT OF THE INVENTION

According to the production method of the O/W emulsion composition of the present invention, an O/W emulsion composition can be easily produced with low energy without using a cooling machine such as an Onlator; thus it is very economical. In addition, the O/W emulsion composition produced by the production method of the present invention has comparable usability to those of the O/W emulsion composition that has the same formulation and is produced with the use of a cooling machine such as an Onlator. Moreover, it is excellent in storage stability.

### MODE FOR CARRYING OUT THE INVENTION

The production method of the O/W emulsion composition of the present invention is characterized by comprising the preparation of an emulsified part that is an highly concentrated O/W emulsion by emulsifying, at a temperature of 70 °C or higher, an oil phase comprising (A) a mono-branched fatty acid POE (0-60) glycerin ester, (B) a linear higher alcohol having 16 or more carbon atoms capable to form an α-gel in water with said (A), and (C) an oil component, and a part of an aqueous phase (a first aqueous phase) comprising (D) water; and the cooling of this emulsified part by mixing with stirring the remaining main aqueous phase (a second aqueous phase) at 10 to 35 °C, wherein an aqueous solvent in the highly concentrated emulsified part is 15 mass % or less. An α-gel is formed in the interface of the emulsion particles of the obtained O/W emulsion composition.

The α-gel is an associated complex consisting of lamellar bimolecular membranes, which are formed by a surfactant with a linear higher alcohol having 16 or more carbon atoms or a non-neutralized fatty acid in the presence of water.

(A) a mono-branched fatty acid POE (0-60) glycerin ester used in the present invention is a nonionic surfactant capable to form an α-gel with (B) a higher alcohol in the aqueous phase. In the present invention, if other nonionic surfactants are used instead of (A) a mono-branched fatty acid POE (0-60) glycerin ester, satisfactory emulsification cannot be achieved in the preparation of the emulsified part even if an α-gel is formed; thus the preparation of the emulsified part itself becomes difficult, and the intended O/W emulsion composition may not be obtained.

(A) a mono-branched fatty acid POE (0-60) glycerin ester (also called "POE (0-60) glyceryl mono-branched fatty acid ester") used in the present invention is a branched fatty acid monoester of polyoxyethylene glyceryl ether with the average addition mole number of oxyethylene groups of 0 to 60. As the branched fatty acid, saturated branched fatty acids having 16 to 24 carbon atoms and preferably having 16 to 20 carbon atoms can be listed. The branching site is not limited in particular. POE represents a polyoxyethylene group, and the average addition mole number is 0 to 60 and preferably 5 to 60. In the present invention, one or more (A) mono-branched fatty acid POE (0-60) glycerin esters can be used.

As one preferable example of (A) a mono-branched fatty acid POE (0-60) glycerin ester, that with the following general formula (1) can be listed. 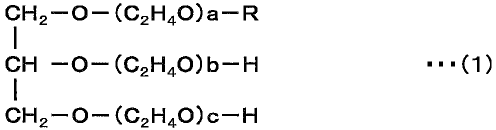 (In the formula, R is the residue after removing an OH group from a saturated branched fatty acid having 16 to 24 carbon atoms or preferably 16 to 20 carbon atoms. a, b, and c are respectively 0 or positive integer, and a+b+c satisfies 0 to 60 and preferably 5 to 60.)

The mono-branched fatty acid POE (0-60) glycerin ester can be easily synthesized by a publicly known method. However, it is convenient if a commercially available nonionic surfactant is used as the nonionic surfactant. Examples include EMALEX GWIS-100 series by Nihon Emulsion Co., Ltd.

As the mono-branched fatty acid POE (0-60) glycerin ester, a hydrophilic one is preferable, and in particular, one with HLB 6 or higher is preferable. In the present invention, a linear fatty acid-type nonionic surfactant such as monostearic acid POE glycerin ester can be used in combination with a mono-branched fatty acid POE (0-60) glycerin ester.

(B) A linear higher alcohol having 16 or more carbon atoms used in the present invention is not limited in particular so far as it can form an α-gel with (A) mono-branched fatty acid POE (0-60) glycerin ester in water and it can be used in the fields of cosmetics, pharmaceuticals, quasi-drugs, etc. Examples thereof include cetyl alcohol, stearyl alcohol, and behenyl alcohol. Preferably, it is a linear saturated higher alcohol having 16 to 24 carbon atoms. In the present invention, one or more (B) linear higher alcohols having 16 or more carbon atoms can be used.

For the formation of α-gel, it is preferable to add batyl alcohol, monoglycerides, etc.

The mass ratio of (A) a mono-branched fatty acid POE (0-60) glycerin ester and (B) a linear higher alcohol having 16 or more carbon atoms, used in the present invention, can be set within the range that the effect of the present invention is not impaired, and it is not limited in particular. Normally, the mass ratio of (A):(B) is 1:2 to 10:1 in the O/W emulsified part.

The concentrations of (A) a mono-branched fatty acid POE (0-60) glycerin ester and (B) a linear higher alcohol having 16 or more carbon atoms are not limited in particular. Preferably, the total amount of (A) the mono-branched fatty acid POE (0-60) glycerin ester and (B) the linear higher alcohol having 16 or more carbon atoms, in the O/W emulsion composition, is 0.5 to 10 parts by mass with respect to 10 parts by mass of (C) the oil component. If it is less than 0.5 parts by mass, a highly stable O/W emulsion composition may not be obtained because of low total amount of (A) and (B). If it exceeds 10 parts by mass, there is an unfavorable trend from the standpoint of usability because of too much total amount of (A) and (B).

(C) An oil component used in the present invention is not limited in particular, and the oil components normally used in cosmetics, pharmaceuticals, etc. can be used.

The solid oil components include oil components that are normally used in cosmetics and external skin preparations and are solid at room temperature. Specific examples thereof include solid fats such as cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, neat's-foot oil, Japan wax, and hydrogenated castor oil; waxes such as beeswax, candelilla wax, cotton wax, carnauba wax, bayberry wax, ibota wax, spermaceti wax, montan wax, rice bran wax, lanolin, kapok wax, acetylated lanolin, sugar cane wax, lanolin fatty acid isopropyl ester, hexyl laurate, hydrogenated lanolin, jojoba wax, lanolin wax, shellac wax, POE lanolin alcohol ether, POE lanolin alcohol acetate, POE cholesterol ether, and POE hydrogenated lanolin alcohol ether; hydrocarbon waxes such as polyethylene wax, paraffin wax, ceresin, petrolatum, microcrystalline wax, Lunacera, and ozokerite; fatty acid glyceryl ethers such as monostearyl glycerin ether (batyl alcohol); fatty acid glycerides such as acetoglyceride and tri-2-heptylundecanoic acid glyceride. These solid oil components can be blended either alone or in combination of two or more.

The liquid oil components include oil components that are normally used in cosmetics and external skin preparations and are liquid at room temperature. Specific examples thereof include liquid fats such as avocado oil, evening primrose oil, camellia oil, turtle oil, macadamia nut oil, sunflower seed oil, almond oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, china paulownia oil, Japanese paulownia oil, jojoba oil, and germ oil; ester oils such as cetyl octanoate, cetyl 2-ethylhexanoate, hexyldecyl dimethyloctanoate, ethyl laurate, hexyl laurate, isopropyl myristate, 2-hexyldecyl myristate, myristyl myristate, octyldodecyl myristate, isopropyl palmitate, 2-ethylhexyl palmitate, 2-hexyldecyl palmitate, 2-heptylundecyl palmitate, butyl stearate, isocetyl stearate, isocetyl isostearate, decyl oleate, dodecyl oleate, oleyl oleate, myristyl lactate, cetyl lactate, diisostearyl malate, cholesteryl 12-hydroxystearate, castor oil fatty acid methyl ester, 2-octyldodecyl N-lauroyl-L-glutamate, 2-ethylhexyl succinate, diisobutyl adipate, 2-hexyldecyl adipate, di-2-heptylundecyl adipate, diisopropyl sebacate, di-2-ethylhexyl sebacate, ethylene glycol di-2-ethylhexanoate, neopentyl glycol dicaprate, neopentyl glycol dioctanoate, acetoglyceride, glyceryl di-2-heptylundecanoate, glyceryl trioctanoate, glyceryl tri-2-ethylhexanoate, glyceryl trimyristate, glyceryl triisopalmitate, glyceryl tri-2-heptylundecanoate, trimethylolpropane tri-2-ethylhexanoate, trimethylolpropane triisostearate, pentaerythrityl tetraoctanoate, and pentaerythrityl tetra-2-ethylhexanoate; hydrocarbon oils such as liquid paraffm, ozokerite, squalene, pristane, and polybutene; unsaturated fatty acid such as oleic acid, linoleic acid, and linolenic acid; branched fatty acids such as isostearic acid, isopalmitic acid, and isomyristic acid; and silicone oils including linear polysiloxanes such as dimethylpolysiloxane, methylphenylpolysiloxane, and diphenylpolysiloxane, cyclic polysiloxanes such as octamethylcyclotetrasiloxane, decamethylcyclopentasiloxane, and dodecamethylcyclohexasiloxane, and various modified polysiloxanes such as amino-modified polysiloxane, polyether-modified polysiloxane, alkyl-modified polysiloxane, and fluorine-modified polysiloxane. These liquid oil components can be blended either alone or in combination of two or more.

In the present invention, it is preferable that one or more higher fatty acids are blended in (C) the oil component. By blending the higher fatty acid these in the oil component, the emulsion particles become much finer, and a more stable O/W emulsion composition can be obtained. As the higher fatty acid, those having 16 to 24 carbon atoms are preferable. Examples thereof include unsaturated fatty acids such as oleic acid, linoleic acid, and linolenic acid; isostearic acid, isopalmitic acid, isomyristic acid, behenic acid, stearic acid, palmitic acid, and myristic acid. The content of higher fatty acids in the O/W emulsion composition is preferably 0.1 to 3 mass % and more preferably 0.2 to 1.5 mass %.

The aqueous phase, comprising (D) water, used in the present invention is that the main medium is water. The hydrophilic components normally used in cosmetics, pharmaceuticals, etc. may be blended thereto within the range that the effect of the present invention is not undermined.

The total blending quantity of water in the O/W emulsion composition of the present invention is not limited in particular. It is generally preferable that the blending quantity is 40 to 95 weight % of the total amount of the O/W emulsion composition.

An aqueous solvent can be blended in the aqueous phase in the O/W emulsion composition of the present invention. However, if the concentration of the aqueous solvent is too high in the emulsified part of the present invention, the preparation of the emulsified part itself may not be achieved. Even if the emulsified part could be prepared, an O/W emulsion composition that has a comparable feeling in use to that of the conventional O/W emulsion composition, which is obtained with the use of an Onlator etc., may not be obtained. In addition, storage stability may be unsatisfactory.

Accordingly, it is preferable that no aqueous solvent is contained in the emulsified part. When an aqueous solvent is contained, the concentration of the aqueous solvent in the emulsified part is preferably 15 mass % or lower, more preferably 3 mass % or lower, and especially preferably 1 mass % or lower.

In cosmetics and external skin preparations, a large amount of aqueous solvent is often blended. The amount of the aqueous solvent in the emulsified part of the present invention is limited as described above; however, the amount of aqueous solvent, which is used for mixing with the emulsified part, in the main aqueous phase (second aqueous phase) is not limited in particular. Thus, the amount of aqueous solvent in the final O/W emulsion composition can be freely designed.

As the aqueous solvent whose blending is limited in the aqueous phase for the preparation of the emulsified part, for example, a water-compatible aqueous solvent that has zero to three hydroxyl groups in the molecule and is liquid at room temperature can be listed. The representative example is a saturated aliphatic polyol having 2 to 3 hydroxyl groups at arbitrary positions. Specific examples include 1,3-butylene glycol, dipropylene glycol, isoprene glycol, 1,2-pentane glycol, 1,2-hexane glycol, 2-methyl-1,3-propane diol, ethyl carbitol, 1,2-butylene glycol, glycerin, etc. Within the range of the present invention, two or more of these can be used in combination.

Among them, glycols (dihydric polyols) especially have a significant effect on the preparation of the emulsified part.

In the production method of the present invention, the main aqueous phase (second aqueous phase) contains (D) water, and the above-described aqueous solvent and other aqueous solvents can be arbitrarily contained. In addition, the components normally used in cosmetics, pharmaceuticals, etc. can also be contained within the range that the effect of the present invention is not impaired.

The amount of the main aqueous phase is preferably 50 to 75 mass % of the total amount of the O/W emulsion composition, and more preferably 55 to 70 mass %. If the amount of the main aqueous phase is less than 50 mass %, a satisfactory cooling effect cannot be obtained, and that much extra energy for cooling is necessary; therefore, it is not economical. If it exceeds 75 mass %, the aqueous phase in the O/W emulsified part, which is prepared at 70 °C or higher, becomes small. Thus, the preparation of a stable O/W emulsion composition tends to become difficult.

The temperature of the main aqueous phase, which is added afterward, is preferably 10 to 35 °C and especially preferably 15 to 30 °C. If the temperature is less than 10 °C, the energy necessary to cool the main aqueous phase becomes excessive, and it tends to be less economical. If the temperature exceeds 35 °C, a satisfactory cooling effect cannot be obtained, and that much extra energy for cooling is necessary; therefore, it is not economical.

It can be confirmed by DSC (differential scanning calorimetry) that the oil phase forms an α-gel in the aqueous phase. The emulsified part obtained by emulsifying the oil phase comprising (A) a mono-branched fatty acid POE (0-60) glycerin ester, (B) a linear higher alcohol having 16 or more carbon atoms, and (C) an oil component in the first aqueous phase is measured by DSC while lowering the temperature to 70 °C or lower. Then an exothermic peak is observed, and this indicates that the oil phase has formed an α-gel in the aqueous phase. In the DSC measurement, other exothermic peaks may be detected in addition to the exothermic peak due to the formation of α-gel. These are exothermic peaks due to simple solidification of components contained in the emulsified part. The exothermic peak due to the formation of α-gel is different from these exothermic peaks due to solidification.

In the present invention, the exothermic peak measurement was carried out with a DSC (Q-2000, TA Instruments, USA) by lowering the temperature at 1 °C/min.

The temperature of the exothermic peak measured with the above-described DSC is considered to correspond to the melting point of the emulsified part in the production method of the present invention.

In the present invention, the emulsified part at 70 °C or higher and the low-temperature main aqueous phase at 10 to 35 °C are mixed with stirring. If the temperature of the mixture immediately after the completion of mixing of the low-temperature main aqueous phase is high, for example 40 °C or higher, the viscosity variation of the obtained O/W emulsion composition may become large. Although the reason for that is not clear, one reason is considered to be immature formation of α-gel. In order to avoid such variation of viscosity, in the present invention, the temperature of the mixture immediately after the completion of mixing of the main aqueous phase and the emulsified part is preferably 35 °C±2 °C or lower. This can be easily controlled by the amount of the main aqueous phase and its temperature.

The O/W emulsion composition prepared by the production method of the present invention is in a state that an associated complex consisting of lamellar bimolecular membranes, which is formed from a mono-branched fatty acid POE (0-60) glycerin ester and a higher alcohol, namely α-gel, is present in the interface of emulsion particles consisting of an oil phase in the presence of water.

Hereinafter, the concept for the production method of the O/W emulsion composition of the present invention will be explained.

### Concept:

An O/W emulsion composition can be obtained by the process in that an oil phase comprising (A) a mono-branched fatty acid POE (0-60) glycerin ester, (B) a linear higher alcohol having 16 or more carbon atoms capable to form an α-gel in water with said (A), and (C) an oil component is emulsified, at 70 °C or higher, with a aqueous phase comprising (D) water, and then the obtained emulsion is cooled.

However, if the emulsion is left to cool, a considerable amount of time is necessary. It is also economically and environmentally disadvantageous if a cooling apparatus such as an Onlator is used.

Accordingly, in order to solve the above-described problem, a highly concentrated emulsified part is prepared at 70 °C or higher (preferably 70 to 80 °C, and more preferably 70 to 75 °C) with the use of a portion of the aqueous phase. The remaining aqueous phase (main aqueous phase) at 10 to 35 °C is gradually mixed to the emulsified part, with stirring. By the mixing of this low-temperature main aqueous phase, the high-temperature emulsified part is rapidly cooled as well as diluted, thus an O/W emulsion composition wherein the α-gel is present in the interface of emulsion particles is prepared. However, if the concentration of the aqueous solvent in the emulsified part is too high in such a method, an O/W emulsion composition excellent in usability and storage stability cannot be obtained. Accordingly, it is necessary in the present invention to let the aqueous solvent in the emulsified part to be 15 mass % or lower. The apparatuses used in emulsification and stirring can suitably be selected from normally used apparatuses. The thus far used apparatuses can be applied, and no special equipment is necessary. The mixing order of the emulsified part and the main aqueous phase is also not limited in particular.

The O/W emulsion composition obtained by the above-described production method can stably exist over a wide temperature range for a long period of time since the particle size is sufficiently small, ranging from 1 to 7.5 µm.

In the conventional production method of an O/W emulsion composition, the following method has been used: water, a moisturizer, and a thickener are dissolved and heated to about 70 °C to prepare a aqueous phase in advance; an oil phase obtained by uniform mixing an oil component, a higher alcohol, and a hydrophilic nonionic surfactant, at about 70 °C, is emulsified into the aqueous phase while being stirred with a homogenizer; and the obtained emulsion is rapidly cooled with an Onlator to about 35 °C. However, the energy use in the conventional production method is wasteful because of heating and the use of a heat exchanger, and the consumption of water used for the cooling machine is large; thus the environmental burden has been high.

On the other hand, according to the production method of the present invention, the O/W emulsion composition can be easily produced with low energy because a cooling machine such as an Onlator is not used when emulsification, and it is not necessary to heat a large amount of aqueous phase. In addition, the emulsification is virtually carried out only with a nonionic surfactant, whose irritation to the human body is relatively small; thus it has excellent safety.

As mentioned above, in the production method of the O/W emulsion composition of the present invention, only by adding the aqueous phase at 10 to 35 °C to the emulsified part produced in advance at 70 to 80 °C, an excellent O/W emulsion composition with the same feeling in use as the conventional one can be obtained. Thus, the conventional production process can be drastically simplified.

The O/W emulsion composition of the present invention can be preferably applied to external preparations used on the body such as the skin and hair in the fields such as cosmetics, pharmaceuticals, and quasi-drugs. For example, it can be used for products such as skin cosmetics, hair cleanser, skin cleanser and hair dressing.

In the O/W emulsion composition of the present invention, in addition to the above-described essential components, the components normally used in cosmetics, pharmaceuticals, etc. can be blended within the range that the effect of the present invention is not undermined. Examples of such components include the following:

Moisturizers such as polyethylene glycol and its mono- or di- alkyl ethers, polyethylene glycol polypropylene glycol copolymer and its mono- or di- alkyl ethers, sorbitol, xylitol, maltitol, mucopolysaccharides, hyaluronic acid, chondroitin sulfate, and chitosan. Thickeners such as methylcellulose, ethylcellulose, gum arabic, and polyvinyl alcohol. Organic solvents such as ethanol. Antioxidants such as butylhydroxytoluene, tocopherol, and phytic acid. Antimicrobial preservative agents such as benzoic acid, salicylic acid, sorbic acid, paraoxybenzoic acid ester (ethylparaben, butylparaben, etc.), and hexachlorophene. Amino acids such as glycine, alanine, valine, leucine, serine, threonine, phenylalanine, tyrosine, aspartic acid, asparagine, glutamine, taurine, arginine, and histidine; and their salts. Organic acids and their salts such as acyl sarcosinic acid (for example, sodium lauroyl sarcosinate), glutathione, citric acid, malic acid, tartaric acid, and lactic acid.

Vitamin A and its derivatives; vitamin Bs such as vitamin B₆ hydrochloride, vitamin B₆ tripalmitate, vitamin B₆ dioctanoate, vitamin B₂ and its derivatives, vitamin B₁₂, and vitamin B₁₅ and its derivatives; vitamin Cs such as ascorbic acid, ascorbyl phosphate ester (its salts), and ascorbyl dipalmitate; vitamin Es such as α-tocopherol, β-tocopherol, γ-tocopherol, vitamin E acetate, and vitamin E nicotinate; vitamin Ds; and vitamins such as vitamin H, pantothenic acid, and pantethine. Various agents such as nicotinic acid amide, benzyl nicotinate, γ-oryzanol, allantoin, glycyrrhizic acid (its salts), glycyrrhetinic acid and its derivatives, hinokitiol, mucidin, bisabolol, eucalyptol, thymol, inositol, saponins (saikosaponin, ginseng saponin, luffa cylindrica saponin, sapindus mukorossi saponin, etc.), pantothenyl ethyl ether, ethinylestradiol, tranexamic acid, cepharanthine, and placenta extract.

Natural extracts obtained by the extraction with a solvent such as organic solvents, alcohols, polyhydric alcohols, water, aqueous alcohols, from materials such as sorrel, Sophora flavescens Aiton, cow lily, orange, sage, thyme, yarrow, mallow, cnidium root, Swertia japonica, Angelica acutiloba, spruce, birch, field horsetail, Luffa cylindrica, horse chestnut, saxifrage, arnica, lily, mugwort, peony, aloe, gardenia, and sawara cypress. Cationic surfactants such as stearyltrimethylammonium chloride, benzalkonium chloride, and laurylamine oxide. Sequestering agents such as disodium edetate, trisodium edetate, sodium citrate, sodium polyphosphate, sodium metaphosphate, and gluconic acid. Neutralizing agents such as potassium hydroxide, sodium hydroxide, and triethanolamine.

In addition, Perfumes, scrubbing agents, powder, coloring agents, whitening agents, UV protection agents such as UV absorbers and UV scattering agents, etc. may be appropriately used so far as effects such as stability is not impaired.

### EXAMPLES

Hereinafter, the present invention will be explained in further detail with specific examples. However, the present invention is not limited by these examples. Unless otherwise noted, the blending quantities are all expressed in mass %.

Initially, the methods for the test and the evaluation used in the present invention will be explained.

### (Viscosity)

With the use of a B-type viscometer (rotor: No. 3, rotation speed: 12 rpm), the viscosity at 30 °C was measured.

### (Storage stability)

After the prepared O/W emulsion composition was stored for one month at 0 °C, 25 °C, and 50 °C, respectively, the composition was evaluated based on the following evaluation criteria under the temperature conditions where the difference in viscosity from that immediately after production is the maximum.
○: Viscosity change is less than 10%.
Δ: Viscosity change is 10% or more and less than 20%.
x: Viscosity change is 20% or more.

Here, Δ or higher was considered as passing.

### (Equivalence to conventional product)

The tests by 10 panelists were carried out, and the number of panelists who answered that the product is equivalent to the conventional product (O/W emulsion composition produced from the same composition with the use of an Onlator) is used as the comprehensive evaluation of the feeling in use (spreadability, blendability, stickiness, freshness, and richness).
○: 8 panelists or more
Δ: 5 panelists or more and 7 panelists or less
X : 4 panelists or less
The evaluation Δ or higher was considered as passing.

**[Table 1]**

| | | | | Production Example | | | | | |
|---|---|---|---|---|---|---|---|---|---|
| | | | | 1-1 | 1-2 | 1-3 | 1-4 | 1-5 | 1-a |
| Emulsified part | Oil phase | Surfactant | PEG-60 glyceryl monoisostearate | 1.3 | 1.3 | 1.3 | 1.3 | 1.3 | 1.3 |
| | | | PEG-5 glyceryl monostearate | 0.7 | 0.7 | 0.7 | 0.7 | 0.7 | 0.7 |
| | | Higher alcohol | Batyl alcohol | 0.15 | 0.15 | 0.15 | 0.15 | 0.15 | 0.15 |
| | | | Behenyl alcohol | 0.3 | 0.3 | 0.3 | 0.3 | 0.3 | 0.3 |
| | | Oil | Behenic acid | 0.3 | 0.3 | 0.3 | 0.3 | 0.3 | 0.3 |
| | | | Stearic acid | 0.4 | 0.4 | 0.4 | 0.4 | 0.4 | 0.4 |
| | | | Isostearic acid | 0.3 | 0.3 | 0.3 | 0.3 | 0.3 | 0.3 |
| | | | Cetyl ethylhexanoate | 3 | 3 | 3 | 3 | 3 | 3 |
| | | | Jojoba seed oil | 1 | 1 | 1 | 1 | 1 | 1 |
| | | | Palm oil | 0.1 | 0.1 | 0.1 | 0.1 | 0.1 | 0.1 |
| | | | Hydrogenated polydecene | 2.5 | 2.5 | 2.5 | 2.5 | 2.5 | 2.5 |
| | | | Dimethylpolysiloxane | 2 | 2 | 2 | 2 | 2 | 2 |
| | | | Perfume | 0.05 | 0.05 | 0.05 | 0.05 | 0.05 | 0.05 |
| | First aqueous phase | | Purified water | 15 | 14 | 11 | 10 | 8 | - |
| | | | Dipropylene glycol | 0 | 1 | 4 | 5 | 7 | - |
| | | | Potassium hydroxide | 0.14 | 0.14 | 0.14 | 0.14 | 0.14 | - |
| Main aqueous phase | Second aqueous phase | | Purified water | 55.96 | 56.96 | 59.96 | 60.96 | 60.96 | 70.96 |
| | | | Dipropylene glycol | 5 | 4 | 1 | 0 | 0 | 5 |
| | | | Potassium hydroxide | - | - | - | - | - | 0.14 |
| | | | Sodium metaphosphate | 0.01 | 0.01 | 0.01 | 0.01 | 0.01 | 0.01 |
| | | | Carboxyvinylpolymer | 0.18 | 0.18 | 0.18 | 0.18 | 0.18 | 0.18 |
| | | | Glycerin | 4 | 4 | 4 | 4 | 4 | 4 |
| | | | Xanthane gum | 0.1 | 0.1 | 0.1 | 0.1 | 0.1 | 0.1 |
| | | | Xylitol | 3 | 3 | 3 | 3 | 3 | 3 |
| | | | Ethanol | 4 | 4 | 4 | 4 | 4 | 4 |
| | | | Phenoxyethanol | 0.5 | 0.5 | 0.5 | 0.5 | 0.5 | 0.5 |
| | | | Sodium pyrosulfite | 0.01 | 0.01 | 0.01 | 0.01 | 0.01 | 0.01 |
| Total amount (%) | | | | 100 | 100 | 100 | 100 | 100 | 100 |
| Aqueous medium in emulsified part(%) | | | | 0 | 3.7 | 14.7 | 18.4 | 25.7 | - |
| Production method | | | | B | B | B | B | B | A |
| Evaluation | | | Viscosity immediately after preparation (mPa·s/30°C) | 5430 | 4560 | 5180 | 4320 | Inability to prepare | 4730 |
| | | | Equivalence to conventional product (Feeling in use) | ○ | Δ | Δ | × | | Standard |
| | | | Storage stability | ○ | Δ | Δ | × | | ○ |

### Production Example A

While the oil phase was being stirred with a homogenizer, the first aqueous phase and the second aqueous phase were added to the oil phase to carry out emulsification at 70 °C. The obtained O/W emulsion was cooled to 35 °C by passing it through an Onlator. Subsequently, it was left to cool to room temperature to obtain an O/W emulsion composition.

### Production Example B

An O/W emulsion (emulsified part) was obtained by emulsifying the oil phase and the first aqueous phase with a homogenizer at 70 °C. This emulsified part was mixed with stirring, by a propeller stirrer (250 rpm), with the main aqueous phase (second aqueous phase) at 25 °C, and an O/W emulsion composition at 35 °C±2 °C was obtained.

All the O/W emulsion compositions obtained in Production Examples 1-1 to 1-5 and Production Example 1-a in Table 1 have the same composition; however, their production methods are different.

Production Example 1-a is an O/W emulsion composition (conventional product) by the conventional method with the use of an Onlator (production method A).

On the other hand, Production Examples 1-1 to 1-5 have the same composition as Production Example 1-a (conventional product). However, all of them are O/W emulsion compositions produced by the cooling method wherein the low-temperature main aqueous phase is mixed without the use of an Onlator (production method B). The amounts of the aqueous solvent (dipropylene glycol) in the emulsified part are different respectively. The emulsified parts in Production Examples 1-1 to 1-5 formed an α-gel.

As seen from Table 1, an O/W emulsion composition having a comparable feeling in use to that of the conventional product was obtained, even without the use of an Onlator, by using a part of the aqueous phase for cooling. However, as seen in Production Examples 1-1 to 1-4, even when the compositions of the final O/W emulsion composition are the same, the concentration of the aqueous solvent in the emulsified part exerts effects on the usability and storage stability of the O/W emulsion composition. As seen in Production Example 1-5, when the concentration of the aqueous solvent in the emulsified part is too high, demulsification takes place in the preparation step of the emulsified part, and the emulsified part itself cannot be prepared.

Thus, in order to obtain an O/W emulsion composition with a comparable feeling in use to that of the conventional product, it is considered that the aqueous solvent in the emulsified part is preferably 15 mass % or less, more preferably 3 mass % or less, and especially preferably 1 mass % or less. Also from the standpoint of storage stability, it is considered to be preferable, as described above, to adjust the concentration of the aqueous solvent in the emulsified part.

**[Table 2]**

| | | | | Production Example | |
|---|---|---|---|---|---|
| | | | | 2-1 | 2-2 |
| Emulsified part | Oil phase | Surfactant | PEG-60 glyceryl monoisostearate | 1.3 | 1.3 |
| | | | PEG-5 glyceryl monostearate | 0.7 | 0.7 |
| | | Higher alcohol | Batyl alcohol | 0.15 | 0.15 |
| | | | Behenyl alcohol | 0.3 | 0.3 |
| | | Oil | Behenic acid | 0.3 | 0.3 |
| | | | Stearic acid | 0.4 | 0.4 |
| | | | Isostearic acid | 0.3 | 0.3 |
| | | | Cetyl ethylhexanoate | 3 | 3 |
| | | | Jojoba seed oil | 1 | 1 |
| | | | Palm oil | 0.1 | 0.1 |
| | | | Hydrogenated polydecene | 2.5 | 2.5 |
| | | | Dimethylpolysiloxane | 2 | 2 |
| | | | Perfume | 0.05 | 0.05 |
| | First aqueous phase | | Purified water | 14 | 5 |
| | | | Glycerin | 1 | 10 |
| | | | Potassium hydroxide | 0.14 | 0.14 |
| Main aqueous phase | Second aqueous phase | | Purified water | 49.96 | 59.96 |
| | | | Dipropylene glycol | 5 | 5 |
| | | | Potassium hydroxide | - | - |
| | | | Sodium metaphosphate | 0.01 | 0.01 |
| | | | Carboxyvinylpolymer | 0.18 | 0.18 |
| | | | Glycerin | 9 | 0 |
| | | | Xanthane gum | 0.1 | 0.1 |
| | | | Xylitol | 3 | 3 |
| | | | Ethanol | 5 | 4 |
| | | | Phenoxyethanol | 0.5 | 0.5 |
| | | | Sodium pyrosulfite | 0.01 | 0.01 |
| Total amount (%) | | | | 100 | 100 |
| Aqueous medium in emulsified part(%) | | | | 3.7 | 36.7 |
| Production method | | | | B | B |
| Evaluation | | | Equivalence to conventional product (Feeling in use) | ○ | Inability to prepare |
| | | | Storage stability | ○ | |

Table 2 shows the results when glycerin was used as the aqueous solvent. Both O/W emulsion compositions obtained in Production Examples 2-1 and 2-2 were produced by the cooling method wherein the low-temperature main aqueous phase is mixed without the use of an Onlator (production method B); the emulsified part formed an α-gel. The equivalence to the conventional product was evaluated by using, as the standard, the O/W emulsion composition of the same composition (conventional product) obtained by the method with the use of an Onlator (production method A).

In Production Example 2-1, an O/W emulsion composition having a comparable feeling in use to that of the conventional product and good in storage stability was obtained. However, as shown in Production Example 2-2, if the concentration of the aqueous solvent in the emulsified part is too high, the emulsified part could not be prepared.

**[Table 3]**

| | | | | Production Example | | | | | |
|---|---|---|---|---|---|---|---|---|---|
| | | | | 3-1 | 3-2 | 3-3 | 3-4 | 3-b | 3-c |
| Emulsified part | Oil phase | Surfactant | Glyceryl isostearate | 0.8 | - | - | - | - | - |
| | | | PEG-8 glyceryl isostearate | - | 0.8 | - | - | - | - |
| | | | PEG-20 glyceryl isostearate | - | - | 0.8 | - | - | - |
| | | | PEG-60 glyceryl isostearate | - | - | - | 0.8 | - | - |
| | | | Glyceryl stearate | - | - | - | - | 0.8 | - |
| | | | POE(30) behenylether | - | - | - | - | - | 0.8 |
| | | | PEG-5 glyceryl monostearate | 1.05 | 1.05 | 1.05 | 1.05 | 1.05 | 1.05 |
| | | Higher alcohol | Behenyl alcohol | 0.5 | 0.5 | 0.5 | 0.5 | 0.5 | 0.5 |
| | | Oil | Glyceryl diisostearate | 0.2 | 0.2 | 0.2 | 0.2 | 0.2 | 0.2 |
| | | | Behenic acid | 0.3 | 0.3 | 0.3 | 0.3 | 0.3 | 0.3 |
| | | | Stearic acid | 0.2 | 0.2 | 0.2 | 0.2 | 0.2 | 0.2 |
| | | | Isostearic acid | 0.3 | 0.3 | 0.3 | 0.3 | 0.3 | 0.3 |
| | | | Hydrogenated polydecene | 3 | 3 | 3 | 3 | 3 | 3 |
| | | | Triethylhexanoin | 3 | 3 | 3 | 3 | 3 | 3 |
| | | | Dimethylpolysiloxane | 2 | 2 | 2 | 2 | 2 | 2 |
| | | | Di(phytosteryl/octyldodecyl) lauroyl glutamate | 0.1 | 0.1 | 0.1 | 0.1 | 0.1 | 0.1 |
| | First aqueous phase | | Purified water | 15 | 15 | 15 | 15 | 15 | 15 |
| | | | Triethanol amine | 0.8 | 0.8 | 0.8 | 0.8 | 0.8 | 0.8 |
| Main aqueous phase | Second aqueous phase | | Purified water | 51.85 | 51.85 | 51.85 | 51.85 | 51.85 | 51.85 |
| | | | Disodium edetate | 0.02 | 0.02 | 0.02 | 0.02 | 0.02 | 0.02 |
| | | | Carboxyvinylpolymer | 0.18 | 0.18 | 0.18 | 0.18 | 0.18 | 0.18 |
| | | | Dipropylene glycol | 5 | 5 | 5 | 5 | 5 | 5 |
| | | | Glycerin | 7 | 7 | 7 | 7 | 7 | 7 |
| | | | Butylene glycol | 7 | 7 | 7 | 7 | 7 | 7 |
| | | | Phenoxyethanol | 0.5 | 0.5 | 0.5 | 0.5 | 0.5 | 0.5 |
| | | | POE(14)POP(7) dimethylether | 0.1 | 0.1 | 0.1 | 0.1 | 0.1 | 0.1 |
| | | | POE(17)POE(4) dimethylether | 0.1 | 0.1 | 0.1 | 0.1 | 0.1 | 0.1 |
| | | | Polyethyleneglycol 1000 | 1 | 1 | 1 | 1 | 1 | 1 |
| Total amount (%) | | | | 100 | 100 | 100 | 100 | 100 | 100 |
| Production method | | | | B | B | B | B | B | B |
| Evaluation | | | Viscosity immediately after preparation (mPa·s/30°C) | 4790 | 2960 | 2870 | 2560 | Inability to prepare | Inability to prepare |
| | | | Equivalence to conventional product (Feeling in use) | ○ | ○ | ○ | ○ | | |
| | | | Storage stability | ○ | ○ | ○ | ○ | | |

Table 3 shows the results obtained in the investigation by changing the types of nonionic surfactants. In each production example, an O/W emulsion composition was produced by the cooling method wherein the low-temperature main aqueous phase is mixed (production method B). All the emulsified parts in Table 3 formed an α-gel. The equivalence to the conventional product was evaluated respectively by using, as the standard, the O/W emulsion composition of the same composition (conventional product) produced according to production method A.

As shown in Table 3, when a mono-branched fatty acid POE (0-60) glycerin ester was used, an O/W emulsion composition having a comparable feeling in use to that of the conventional product was obtained, and they were excellent in storage stability (Production Examples 3-1 to 3-4).

On the other hand, when other nonionic surfactants such as mono-linear fatty acid (POE) glycerin esters and POE alkyl ethers were used instead of a mono-branched fatty acid POE (0-60) glycerin ester, in the preparation step of an emulsified part, satisfactory emulsification could not be achieved, the emulsified part separated out or separated from water, and the intended O/W emulsion composition could not be obtained (Production Example 3-b, Production Example 3-c).

Thus, it is understood in the present invention that the use of a mono-branched fatty acid POE (0-60) glycerin ester is important.

### Example 1 Milky lotion

### <Second aqueous phase (main aqueous phase)>

| Purified water | balance |
|---|---|
| Tranexamic acid | 2.0 mass % |
| Disodium edetate | 0.01 |
| Xylitol | 3.0 |
| Glycerin | 3.0 |
| Dipropylene glycol | 7.0 |
| Xanthan gum | 0.05 |
| Carboxyvinyl polymer | 0.1 |
| Ethanol | 3.0 |
| Phenoxyethanol | 0.5 |
| <Oil phase> | |
| Cetyl ethylhexanoate | 4.0 |
| Liquid paraffin | 3.5 |
| PEG-60 glyceryl monoisostearate | 1.0 |
| PEG-5 glyceryl monostearate | 1.0 |
| Behenic acid | 0.3 |
| Stearic acid | 0.4 |
| Isostearic acid | 0.3 |
| Behenyl alcohol | 0.3 |
| Batyl alcohol | 0.15 |
| Vaseline | 0.5 |
| Dimethylpolysiloxane | 1.0 |

### <First aqueous phase (for preparing emulsified part)>

| | |
|---|---|
| Potassium hydroxide | 0.08 |
| Purified water | 15.0 |

### Production method:

An O/W milky lotion was obtained by a method based on production method B. The viscosity of the milky lotion was 4,370 mPa·s/30 °C. This milky lotion had a comparable feeling in use to that of the conventional product of the same composition (O/W milky lotion obtained by production method A wherein cooling is done with an Onlator etc.), and the storage stability was also excellent.

### Example 2 Milky lotion

### <Second aqueous phase (main aqueous phase)>

| Purified water | balance |
|---|---|
| Potassium 4-methoxysalicylate | 1.0 mass % |
| Disodium edetate | 0.05 |
| Glycerin | 7.0 |
| Dipropylene glycol | 7.0 |
| Butylene glycol | 5.0 |
| Succinoglycane | 0.04 |
| Carboxyvinyl polymer | 0.15 |
| Ethanol | 1.0 |
| Phenoxyethanol | 0.5 |
| Dipotassium glycyrrhizinate | 0.05 |
| <Oil phase> | |
| Triethylhexanoin | 4.0 |
| Hydrogenated polydecene | 6.0 |
| PEG-60 glyceryl monoisostearate | 1.2 |
| PEG-5 glyceryl monostearate | 0.8 |
| Behenic acid | 0.45 |
| Stearic acid | 0.6 |
| Isostearic acid | 0.45 |
| Behenyl alcohol | 0.95 |
| Batyl alcohol | 0.25 |
| Vaseline | 2.0 |
| Glyceryl diisostearate | 2.0 |
| Di(phytosteryl/octyldodecyl) lauroyl glutamate | 0.1 |
| Dimethylpolysiloxane | 2.0 |
| Methylphenylpolysiloxane | 1.5 |
| <First aqueous phase (for preparing emulsified part)> | |
| Triethanolamine | 1.5 |
| Purified water | 15.0 |

### Production method:

An O/W milky lotion was obtained by a method based on production method B. The viscosity of the milky lotion was 3,690 mPa·s/30 °C. This milky lotion had a comparable feeling in use to that of the conventional product of the same composition (O/W milky lotion obtained by production method A wherein cooling is done with an Onlator etc.), and the storage stability was also excellent.

### Example 3 Milky lotion

### <Second aqueous phase (main aqueous phase)>

| Purified water | balance |
|---|---|
| Sodium metaphosphate | 0.01 mass % |
| Xylitol | 3.0 |
| Glycerin | 4.0 |
| Dipropylene glycol | 5.0 |
| Xanthan gum | 0.1 |
| Carboxyvinyl polymer | 0.18 |
| Ethanol | 4.0 |
| Phenoxyethanol | 0.5 |

### <Oil phase>

| | |
|---|---|
| Cetyl ethylhexanoate | 3.0 |
| Hydrogenated polydecene | 3.5 |
| Dimethylpolysiloxane | 2.0 |
| PEG-60 glyceryl monoisostearate | 1.3 |
| PEG-8 glyceryl monoisostearate | 0.7 |
| Behenic acid | 0.3 |
| Stearic acid | 0.4 |
| Isostearic acid | 0.3 |
| Behenyl alcohol | 0.3 |
| Batyl alcohol | 0.15 |

### <First aqueous phase (for preparing emulsified part)>

| | |
|---|---|
| Potassium hydroxide | 0.14 |
| Purified water | 15.0 |

### Production method:

An O/W milky lotion was obtained by a method based on production method B. The viscosity of the milky lotion was 3,260 mPa·s/30 °C. This milky lotion had a comparable feeling in use to that of the conventional product of the same composition (O/W milky lotion obtained by production method A wherein cooling is done with an Onlator etc.), and the storage stability was also excellent.

## Claims

1. A production method of an O/W emulsion composition comprising the preparation of an emulsified part that is an O/W emulsion by emulsifying, at a temperature of 70 °C or higher, an oil phase comprising
(A) a mono-branched fatty acid POE (0-60) glycerin ester,
(B) a linear higher alcohol having 16 or more carbon atoms capable to form an α-gel in water with said (A), and
(C) an oil component,
and a part of an aqueous phase (a first aqueous phase) comprising (D) water;
and the cooling of this emulsified part by mixing with stirring the remaining main aqueous phase (a second aqueous phase) at 10 to 35 °C, wherein an aqueous solvent in the emulsified part is 15 mass % or less.

2. The production method of an O/W emulsion composition according to claim 1, wherein the aqueous solvent is a dihydric to trihydric polyol.

3. The production method of an O/W emulsion composition according to claim 1 or 2, wherein the main aqueous phase is 50 to 75 mass % of the total O/W emulsion composition.

4. The production method of an O/W emulsion composition according to any one of claims 1 to 3, wherein the sum of (A) a mono-branched fatty acid POE (0-60) glycerin ester and (B) a linear higher alcohol having 16 or more carbon atoms is 0.5 to 10 parts by mass with respect to 10 parts by mass of (C) an oil component.
